Europäisches Patentamt

European Patent Office

Office européen des brevets

(11) Publication number: **0 400 471**
**A1**

# EUROPEAN PATENT APPLICATION

(21) Application number: 90109804.6

(22) Date of filing: 23.05.90

(51) Int. Cl.⁵: **A61B 17/36**

(30) Priority: 30.05.89 JP 134670/89

(43) Date of publication of application:
05.12.90 Bulletin 90/49

(84) Designated Contracting States:
**DE FR SE**

(71) Applicant: **Kabushiki Kaisha TOPCON**
**75-1, Hasunuma-cho Itabashi-ku**
**Tokyo(JP)**

(72) Inventor: **Tomioka, Yuko**
**75-1 Hasunuma-cho**
**Itabashi-ku - Tokyo(JP)**
Inventor: **Koizumi,Hirishi**
**75-1 Hasunuma-cho**
**Itabashi-ku,Tokyo(JP)**
Inventor: **Kijima, Masatsugu**
**75-1 Hasunuma-cho**
**Itabashi-ku,Tokyo(JP)**

(74) Representative: **Witte, Alexander, Dr.-Ing. et al**
**Augustenstrasse 7**
**D-7000 Stuttgart 1(DE)**

(54) Laser treatment apparatus.

(57) A laser treatment apparatus for treating a site of treatment of a patient with laser beams contains a memory unit (13) for storing a plurality of conditions for applying the laser beams to the site of' treatment of the patient; and a control unit (10) for applying the laser beams so as to be suitable for treatment of the site of treatment on the basis of the conditions stored by the memory unit (13).

Fig. 1

## BACKGROUND OF THE INVENTION

### 1. Field of the Invention

The present invention relates to a laser treatment apparatus for treating a patient by applying laser light to a site of treatment of the patient.

### 2. Description of Related Art

Conventional laser treatment apparatuses require individual operation switches to set a plurality of conditions such as a time period for applying a laser beam, power of the laser light and the size of a spot of the laser beam prior to the start of treatment. Thus, whenever the object of treatment such as a disease to be treated or the state of disease changes, the setting of new conditions should be made by operating such individual switches. For a photocoagulator for optical surgery, for instance, the time period for applying the laser beams should be set to 200 milliseconds, the power to 200 mW and the spot size to 200 microns in the case of treatment of diabetic retinitis, on the one hand, and the time period for applying the laser beams should be set to 20 milliseconds, the power to 1 W and the spot size to 50 microns in the case of glaucome.

Therefore, when treatment of glaucome should be made after treatment of diabetic retinitis, all the settings for treatment of diabetic retinitis should be changed to the settings for treatment of glaucome. When the changes are required to such a great extent, the switches are usually turned several times, thereby presenting the difficulty of setting the conditions in a short time period and leading to the likelihood to cause an error in the setting.

## SUMMARY OF THE INVENTION

Therefore, the present invention has the object to provide a laser treatment apparatus adapted to allow a quick change in the setting.

In order to achieve the object, the present invention consists of a laser treatment apparatus for treating a site of treatment of a patient with laser beams, comprising:
a memory unit for storing a plurality of conditions for applying the laser beams to the site of treatment of the patient; and
a control unit for applying the laser beams so as to be suitable for treatment of the site of treatment on the basis of the conditions stored by the memory unit.

## BRIEF DESCRIPTION OF THE DRAWINGS

Other objects, features and advantages of the present invention will become apparent in the course of the description of the preferred embodiments which follows in conjunction with the accompanying drawings.

FIG. 1 is a side view of the laser treatment apparatus according to an embodiment of the present invention with a block diagram showing a basic construction thereof.

FIGS. 2 and 3 are flow charts showing procedures of treatment to be performed by the laser treatment apparatus according to an embodiment of the present invention.

## DETAILED DESCRIPTION OF THE PREFERRED EMBODIMENTS

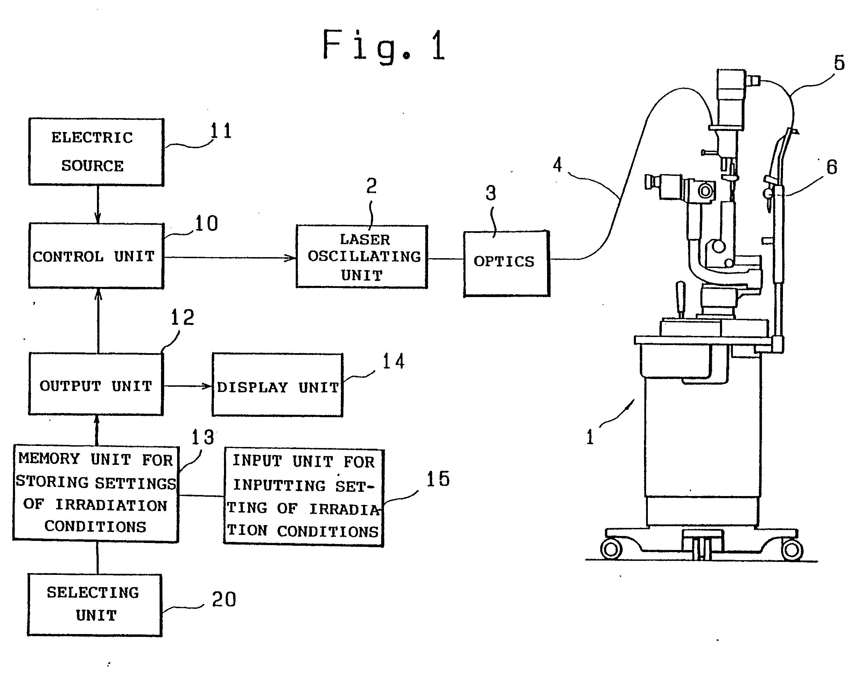

As shown in FIG. 1, a memory unit 13 is designed so as to store a plurality of conditions for applying laser beams to a site of treatment for treating the site of treatment, and a control unit 10 is constructed so as to irradiate the site of treatment with the laser beams on the basis of the conditions stored by the memory unit.

It is appreciably noted that the laser treatment apparatus according to the present invention can perform surgery on the conditions stored in the memory unit even if changes in treatment are to be performed.

Referring to FIG. 1, the laser treatment apparatus according to the present invention is designed such that laser beams are applied to the site of treatment to be subjected to surgery.

An operation unit 1 comprises a photocoagulator for optical surgery which has a slit 1amp which is used to three-dimensionally observe the eyeground of the eye of a person undergoing examination with high magnifications. The operation unit 1 is further provided with a laser oscillating unit 2 connected through optics 3 to optical fibers 4. The optics 3 may comprise a pumping lens, for example, and the laser light irradiated from the laser oscillating unit 2 is led to the operation unit 1 through the optical fibers 4. The laser light is applied to the site of treatment through the optical fibers 4 and a handpiece 6 which is operable by the operator.

The laser oscillating unit 2 is to treat the site of eye treatment and the laser light from the laser oscillating unit 2 is used, for instance, for photocoagulating the site of treatment in the eyeground. As the laser oscillating unit 2 may be used, for example, argon laser, krypton laser, argon/krypton laser, argon/dye lasers, and so on. Among these lasers, the argon/krypton laser and argon/dye lasers are variable in wavelength. The argon/krypton laser has three wavelengths of 488 + 514.5 nm, 514.5 nm and 647 nm, while the argon/dye laser has multiple wavelengths of 488 + 514.5 nm, 514.5 nm and 577-630 nm. The particular wavelength may be used so as to comply with the site of treatment.

Recent years, attention has increasingly been paid particularly to the argon/dye lasers, among others, due to the effectiveness of the lasers ranging from yellow to orange in color and having wavelengths ranging from 577 nm to 600 nm for treatment of macula which is said to be of the most significance for the eyeground of the eye .

To the laser oscillating unit 2 is connected a control unit 10 which in turn is connected to a source of electricity 11 and an output unit 12. The output unit 12 is further connected to the memory unit 13 and a display unit 14. The conditions for applying the laser beams from an input unit 15 for inputting the setting of the conditions for applying the laser beams are stored in advance in the memory unit 13.

The conditions as stored in advance by the memory unit 13 are displayed on the display unit 14 such as a CRT display, in addition to generation to the output unit 12. The control unit 10 is designed to control the laser beam oscillating unit 2 in accordance with the irradiating conditions from the output unit 12.

The irradiating conditions may comprise at least a quantity of laser beam generated, a period of time during which the laser beam is applied, and the size of a spot of the laser beam applied. These three conditions may vary with the purpose of treatment. For instance, for treatment of diabetic retinitis, the time period may be 200 milliseconds, the power may be 200 mW, and the spot size may be 200 microns. For the treatment of glaucome, the time period may be 20 milliseconds, the power may be 1 W, and the spot size may be 50 microns.

Conditions of applying the laser beam for treatment of a plurality of diseases, such as diabetic retinitis and glaucome, may be stored in advance in the memory unit 13 from the input unit 15 for inputting the setting of the irradiating conditions.

The operator operates a selecting unit 20 to thereby give an instruction of desired irradiating conditions stored in the memory unit 13 to the laser light oscillating unit 2.

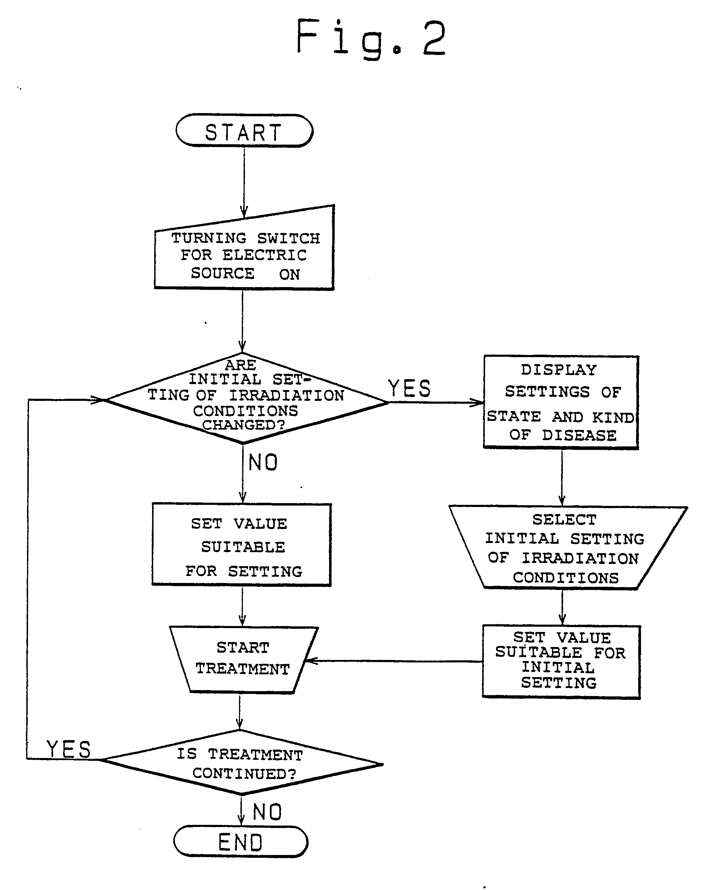

FIG. 2 shows the flow chart showing the case of treating one kind of disease.

Referring to FIG. 2, the operator turns a electric source 11 of FIG. 1 on to selectively set initial conditions for irradiation so as to correspond to the kind of disease and a state of the disease. In other words, the operator operates the selecting unit 20, thereby generating the irradiating conditions suitable for treatment from the memory unit 13 to the output unit 12. The laser oscillating unit 2 is then operated in an appropriate manner on the basis of the irradiating conditions instructed from the output unit 12 to the control unit 10. This arrangement permits an accurate and sure operation.

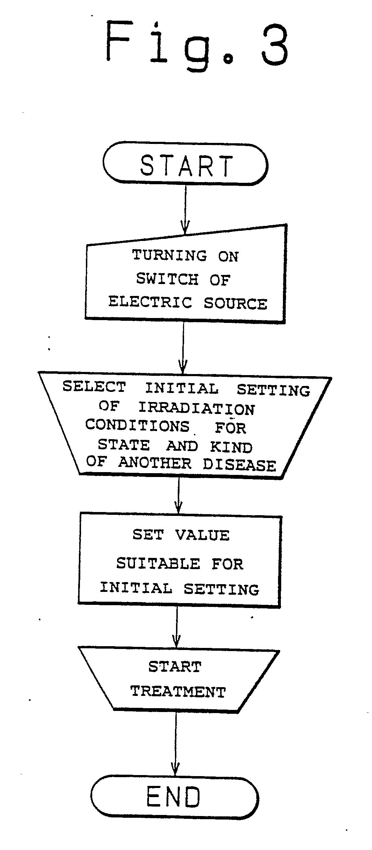

FIG. 3 shows the flow chart showing the case of changing treatment from one disease to another disease.

The operator turns the electric source 11 of FIG. 1 on to selectively change the setting of the initial conditions for irradiation by displaying the irradiating conditions on the display unit 14 from the irradiating conditions of all the states and kinds of diseases stored in the memory unit 13. The operator can select the necessary irradiating conditions by means of the selecting unit 20 while confirming the conditions displayed on the display unit 14. For instance, the irradiating conditions for treatment of retinitis may be changed to those for treatment of glaucome, thereby permitting treatment of glaucome after completion of treatment of retinitis with the laser beams on the basis of the irradiating conditions newly set to correspond to glaucome.

It is further to be noted that, when the laser treatment apparatus according to the present invention is used for treatment of another disease, new conditions for irradiation with laser beams can be selected in the same manner as described hereinabove.

As a matter of course, when the same disease is treated by the laser treatment apparatus according to the present invention, no changes in the irradiating conditions can be required.

As have been described hereinabove, the laser treatment apparatus according to the present invention permits treatment of disease by reading irradiating conditions stored in advance so as to correspond to the purpose for treatment by surgery. The laser treatment apparatus according to the present invention saves laborious work that has otherwise been required by conventional apparatuses by changing the irradiating conditions. It can further prevent an error in irradiation with laser beams and improve safety of surgery.

The present invention may be embodied in other specific forms without departing from the spirit and scope thereof. The present embodiments as described hereinabove are therefore to be considered in all respects as illustrative and not restrictive, the scope of the invention being indicated by the appended claims, and all the changes, modifications and variations which come within the meaning and range of equivalency of the claims are therefore intended to be encompassed within the spirit and scope of the invention.

## Claims

1. A laser treatment apparatus for treating a site of treatment of a patient with laser beams, comprising:
- a memory unit (13) for storing a plurality of conditions for applying the laser beams to the site of treatment of the patient; and
- a control unit (10) for applying the laser beams so as to be suitable for treatment of the site of treatment on the basis of the conditions stored by the memory unit (13).

2. A laser treatment apparatus according to claim 1, characterized by further comprising an operation unit (1).

3. A laser treatment apparatus according to claim 2, characterized in that the operation unit (1) has a laser oscillating unit (2).

4. A laser treatment apparatus according to claim 2 or 3, characterized in that the operation unit (1) is a photocoagulator for opthalmology.

5. A laser treatment apparatus according to claims 3 or 4, characterized in that the laser oscillating (2) unit is optically connected to optics (3).

6. A laser treatment apparatus according to claim 5, characterized in that the optics (3) is a pumping lens.

7. A laser treatment apparatus according to any one of claims 3 through 5, characterized in that the laser oscillating (2) unit generates a laser beam.

8. A laser treatment apparatus according to claim 7, characterized in that the laser beam is an argon laser, a krypton laser, an argon/krypton laser or an argon/dye laser.

9. A laser treatment apparatus according to any one of claims 1 through 8, characterized in that the control unit (10) is connected to a laser oscillating unit (2).

10. A laser treatment apparatus according to any one of claims 1 through 9, characterized in that the conditions comprise a period of time during the laser beam is irradiated, a power of the laser beam and a size of a spot of the laser beam.

Fig. 1

ELECTRIC SOURCE 11

CONTROL UNIT 10

OUTPUT UNIT 12

MEMORY UNIT FOR STORING SETTINGS OF IRRADIATION CONDITIONS 13

SELECTING UNIT 20

LASER OSCILLATING UNIT 2

OPTICS 3

DISPLAY UNIT 14

INPUT UNIT FOR INPUTTING SETTING OF IRRADIATION CONDITIONS 15

4

5

6

1

# Fig. 2

# Fig. 3

```
         ┌─────────────┐
         │    START     │
         └──────┬───────┘
                │
                ▼
        ╱───────────────╲
       │   TURNING ON     │
       │   SWITCH OF       │
       │  ELECTRIC SOURCE  │
        └─────────┬────────┘
                  │
                  ▼
       ╱─────────────────────╲
      │  SELECT  INITIAL  SETTING │
      │    OF  IRRADIATION        │
      │  CONDITIONS  FOR          │
      │    STATE  AND KIND        │
       ╲  OF ANOTHER DISEASE     ╱
        └──────────┬───────────┘
                   │
                   ▼
         ┌──────────────────┐
         │    SET VALUE       │
         │  SUITABLE FOR      │
         │  INITIAL SETTING   │
         └────────┬──────────┘
                  │
                  ▼
         ╱──────────────╲
        │    START        │
        │  TREATMENT      │
         └───────┬───────┘
                 │
                 ▼
         ┌──────────────┐
         │     END       │
         └──────────────┘
```

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 403 220 (PROMED TECHNOLOGY) * Abstract; page 7, line 21 - page 8, line 20; page 15, lines 27-35 * | 1-10 | A 61 B 17/36 |
| X | FR-A-2 513 028 (NIPPON INFRARED INDUSTRIES) * Page 1, line 1 - page 3, line 6; page 11, lines 1-23 * | 1-3,5-7 ,9,10 | |

TECHNICAL FIELDS SEARCHED (Int. Cl.5)

A 61 B
A 61 F

The present search report has been drawn up for all claims

| Place of search | Date of completion of the search | Examiner |
|---|---|---|
| THE HAGUE | 22-08-1990 | WOLF C.H.S. |